# EUROPEAN PATENT APPLICATION

(11) **EP 3 427 774 A1**
(43) Date of publication of application: **16.01.2019**
(21) Application number: 18182611.6
(22) Date of filing: 10.07.2018
(51) Int. Cl.: A61M 1/28, A61D 7/00

(54) **PERITONEAL DIALYSIS DEVICE FOR ANIMALS**

(30) Priority: 11.07.2017 TW 10610194 U
(71) Applicant: Benepet Co., Ltd., Taipei City 10489 (TW)
(72) Inventor: Tan, Ta-Lun, Taipei City (TW)
(74) Representative: Swindell & Pearson Limited

(57) **Abstract**

The present invention relates to a peritoneal dialysis device for animals, comprising: a dialysate catheter, one end of which is connected to a dialysate container connector and the other end of which is connected to a four-way joint; an injection catheter, one end of which is connected to an injection catheter connector and the other end of which is connected to the four-way joint; a waste fluid catheter, one end of which is connected to a waste fluid container connector and the other end of which is connected to the four-way joint; and a syringe catheter, one end of which is connected to a syringe and the other end of which is connected to the four-way joint. The present invention may accurately quantify the amount of the dialysate injected, slowly but continuously and stably inject the dialysate, and automatically inject the dialysate without the need for human care worker to perform and monitor personally, thus saving a lot of manpower.

## Description

### BACKGROUND

### Technical Field

The present invention relates to a peritoneal dialysis device for animals, and more particularly to a peritoneal dialysis device for animals which can accurately, stably and automatically inject the dialysate.

Peritoneal dialysis is a therapy method for clearing metabolic wastes in the blood of patients with renal failure. The method is performed by injecting dialysate into the peritoneal cavity of the patients with renal failure for a period of time and using the peritoneum as a dialysis semipermeable membrane to remove and transport the body wastes and water to the dialysate in the peritoneal cavity, and then draining out the waste fluid containing the body wastes and water, to achieve the purpose of clearing the metabolism wastes in the blood. When practicing the peritoneal dialysis, the patients have to repeat practicing the skill of fluid injection and exchange while learning dialysis-related care, before they can independently perform home peritoneal dialysis therapy.

However, when applying peritoneal dialysis to animals, a variety of difficulties are encountered. (1) Due to different sizes of animals, the amount of dialysate to be injected is not the same. Particularly, small animals are very sensitive to the amount of dialysate injected, which may cause harm or even death to the animal as long as there is an error in the injection amount. In addition, animals are not able to express their own feelings and inform the care worker when they feel discomfort, which further increases the accuracy requirement of the amount of the dialysate injected. The traditional peritoneal dialysis device for human body can not accurately quantify the amount of the dialysate injected, which may easily cause harm to animals. (2) As smaller animal has smaller peritoneal cavity and requires smaller amount of dialysate injected, applying the traditional peritoneal dialysis device for human body on small animal and pushing the dialysate only by gravity, would make the dialysate flow unstable or even stagnant. (3) Due to the animal's inability to perform peritoneal dialysis on its own, the peritoneal dialysis must be performed and supervised by human care workers, thus consuming a great deal of manpower.

Therefore, it is necessary to develop a device to solve the above problems.

### SUMMARY

The object of the present invention is to provide a peritoneal dialysis device for animals which can (1) accurately quantify the amount of the dialysate injected, (2) slowly but continuously and stably inject the dialysate, and (3) automatically inject the dialysate without the need for human care worker to perform and supervise personally, thus saving a lot of manpower.

The technical solutions of the present invention for solving the above technical problems are as follows:
a dialysate catheter, one end of which is connected to a dialysate container connector and the other end of which is connected to a four-way joint;
an injection catheter, one end of which is connected to an injection catheter connector and the other end of which is connected to the four-way joint;
a waste fluid catheter, one end of which is connected to a waste fluid container connector and the other end of which is connected to the four-way joint; and
a syringe catheter, one end of which is connected to a syringe and the other end of which is connected to the four-way joint.

In one embodiment of the present invention, the dialysate catheter is connected to a dialysate container through the dialysate container connector; the injection catheter is connected to a peritoneal catheter through the injection catheter connector; and the waste fluid catheter is connected to a waste fluid container through the waste fluid container connector.

In one embodiment of the present invention, each of the dialysate container connector, the injection catheter connector and the waste fluid container connector is a luer taper.

In one embodiment of the present invention, the injection catheter comprises a waterwheel that rotates in response to the flow of fluid in the injection catheter.

In one embodiment of the present invention, the waste fluid catheter comprises a sampling port.

In one embodiment of the present invention, the dialysate catheter comprises a dialysate catheter clamp disposed on the dialysate catheter and configured to open or close the passageway of the dialysate catheter; the injection catheter comprises an injection catheter clamp disposed on the injection catheter and configured to open or close the passageway of the injection catheter; the waste fluid catheter comprises a waste fluid catheter clamp disposed on the waste fluid catheter and configured to open or close the passageway of the waste fluid catheter.

In one embodiment of the present invention, the dialysate catheter comprises a multi-way joint including three or more ends, wherein two or more ends are connected to a plurality of the dialysate container connectors, and the other one end is connected to the four-way joint.

In one embodiment of the present invention, the dialysate catheter is connected to a plurality of dialysate containers through the plurality of the dialysate container connectors.

In one embodiment of the present invention, the injection catheter connector is connected to the peritoneal catheter through an isolating short tube, so as to prevent the injection catheter connector from being directly connected to the peritoneal catheter.

In the peritoneal dialysis device for animals of the present invention, the dialysate is drawn up into the syringe before it is injected into the peritoneum, and the amount of the dialysate is precisely quantified on the scale of the syringe, so as to overcome the low accuracy of the conventional peritoneal dialysis devices. Then the care worker or machine slowly but stably injects the dialysate of the syringe into the peritoneal cavity through the catheters and connectors by applying a steady force to the plunger of the syringe, so as to overcome the instability problems of conventional peritoneal dialysis devices which rely on gravity to push the fluid in the catheter. When the syringe is installed on a machine which can apply a stable force to push the plunger of the syringe slowly and at a constant speed, the peritoneal dialysis device for animals according to the present invention can automatically inject the dialysate without the need for human care worker to perform and supervise personally, thus saving a lot of manpower.

### BRIEF DESCRIPTION OF DRAWINGS

To more clearly describe the technical solutions of the embodiments of the present invention or of the prior art, the drawings required for describing the embodiments or the prior art will be briefly described in the following context. Apparently, the drawings in the following description are merely some embodiments of the present invention, and the person of ordinary skill in the art may derive other drawings from these drawings without creative efforts.
Fig. 1 is a schematic diagram of the peritoneal dialysis device for animals according to one embodiment of the present invention.

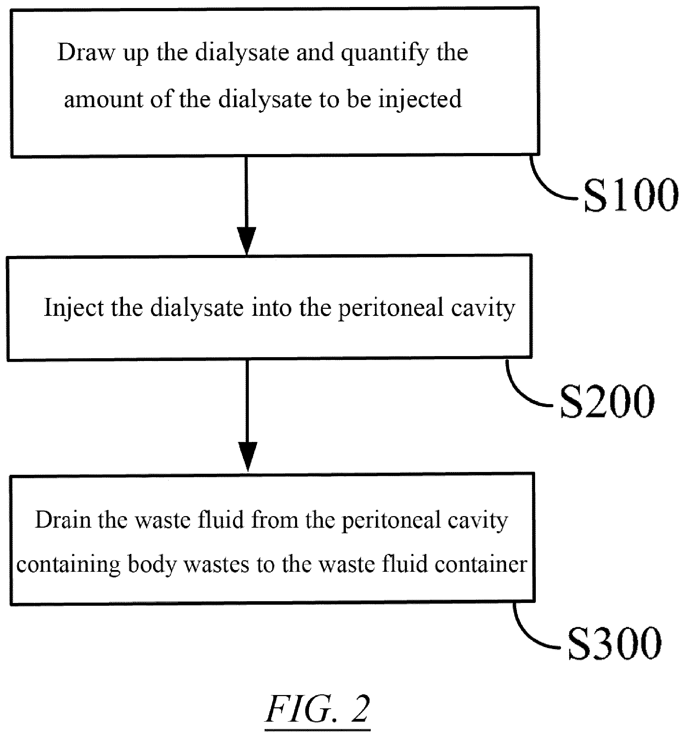

Fig. 2 is a flow chart of the method for using the peritoneal dialysis device for animals according to one embodiment of the present invention.

### Explanation of references:

- 1: peritoneal dialysis device for animals
- 10: dialysate catheter
- 11: dialysate container connector
- 12: dialysate catheter clamp
- 13: multi-way joint
- 20: injection catheter
- 21: injection catheter connector
- 22: injection catheter clamp
- 23: waterwheel
- 24: peritoneal catheter
- 25: isolating short tube
- 30: waste fluid catheter
- 31: waste fluid container connector
- 32: waste fluid catheter clamp
- 33: waste fluid container
- 34: sampling port
- 40: syringe catheter
- 41: syringe
- 50: four-way connector

### DETAILED DESCRIPTION

The present invention will be further described in detail with reference to the embodiments. The following embodiments are intended to explain the present invention, but the present invention is not limited to the following embodiments.

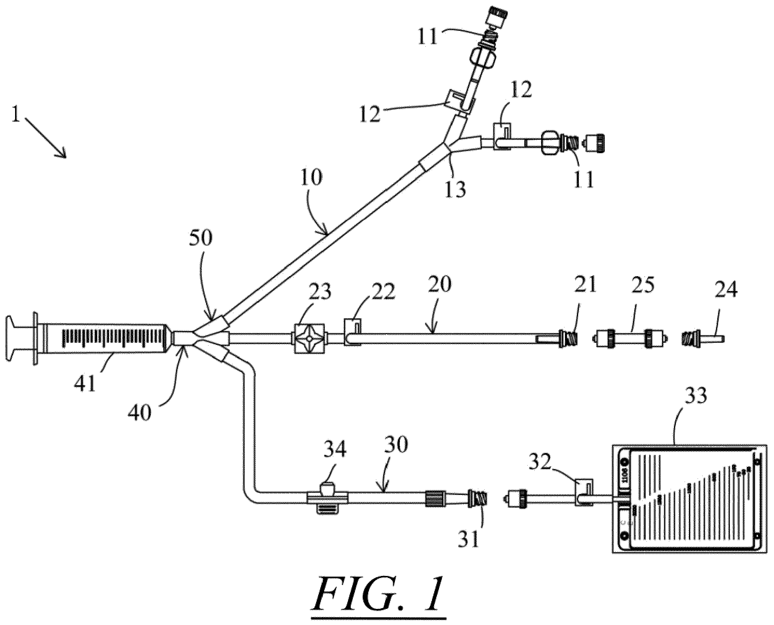

Referring to Fig. 1, Fig.1 is a schematic diagram of the peritoneal dialysis device for animals according to one embodiment of the present invention. The present invention provides a peritoneal dialysis device for animals 1, comprising a dialysate catheter 10, an injection catheter 20, a waste fluid catheter 30, and a syringe catheter 40. One end of the dialysate catheter 10 is connected to a dialysate container connector 11 and the other end of the dialysate catheter 10 is connected to a four-way joint 50. One end of the injection catheter 20 is connected to an injection catheter connector 21 and the other end of the injection catheter 20 is connected to the four-way joint 50. One end of the waste fluid catheter 30 is connected to a waste fluid container connector 31 and the other end of the waste fluid catheter 30 is connected to the four-way joint 50. One end of the syringe catheter 40 is connected to a syringe 41 and the other end of the syringe catheter 40 is connected to the four-way joint 50.

The dialysate catheter 10 is used for transporting the dialysate flowing out from a dialysate container to the direction of the syringe 41. The dialysate catheter 10 is connected to a dialysate container (not shown in the figure) through the dialysate container connector 11, and the dialysate flowing out from the dialysate container sequentially passes through the dialysate container connector 11, the dialysate catheter 10, the four-way joint 50 and the syringe catheter 40 before entering into the syringe 41.

In one embodiment of the present invention, the dialysate container connector 11 is a luer taper. In the peritoneal dialysis device for animals 1 shown in Fig. 1, the portion of the dialysate container connector 11 connected to the dialysate catheter 10 is a male connector; and the portion of the dialysate container connector 11 connected to the dialysate container 10 is a female connector. In fact, the portion of the dialysate container connector 11 connected to the dialysate catheter 10 may also be a female connector, and the portion of the dialysate container connector 11 connected to the dialysate container 10 may also be a male connector. However, which one is male connector or female connector, is merely an embodiment of the present invention, and should not be conceived to limit the scope of this patent application. In one embodiment of the present invention, the dialysate catheter 10 further comprises a dialysate catheter clamp 12 disposed on the dialysate catheter 10 and configured to open or close the passageway of the dialysate catheter 10. However, it may not be necessary to use the catheter clamp 12 to open or close the passageway of the dialysate catheter 10. The catheter may also be manually squeezed by a machine or a care worker to close the passageway of the dialysate catheter 10, or controlled by a valve disposed inside the dialysate catheter 10 to open or close the passageway of the catheter 10 (not shown).

In one embodiment of the present invention, the dialysate catheter 10 comprises a multi-way joint 13 configured to connect a plurality of dialysate catheter branches. The multi-way joint 13 comprises three or more ends, wherein two or more ends are connected to a plurality of the dialysate container connectors 11, and the other one end is connected to the four-way joint 50, as shown in Fig. 1. The dialysate catheter 10 is connected to a plurality of dialysate containers (not shown in the figure) through the plurality of the dialysate container connectors 11. Thus the peritoneal dialysis device for animals 1 of the present invention may be connected to a plurality of dialysate containers. As soon as the dialysate in one dialysate container is used up, another dialysate container will back up.

The injection catheter 20 is used for transporting the dialysate flowing out from the syringe 41 to the direction of the peritoneum. The injection catheter 20 is connected to a peritoneal catheter 24 through the injection catheter connector 21. The peritoneal catheter 24 is a catheter of which one end is implanted into the peritoneal cavity of the patient, for introducing external dialysate into the peritoneal cavity of the patient, allowing the dialysate to contact the patient's peritoneum for a period of time. Using the peritoneum as a dialysis semipermeable membrane, the body wastes are removed and transported to the peritoneal dialysate, and thereafter the waste fluid containing the body wastes is drained out through the peritoneal catheter 24. The dialysate from the syringe 41 sequentially passes through the syringe catheter 40, the four-way joint 50, the injection catheter 20, the injection catheter connector 21 and the peritoneal catheter 24 before entering into the peritoneal cavity of the patient.

In one embodiment of the present invention, the injection connector 21 is a luer taper. Since one end of the peritoneal catheter 24 is directly implanted inside the peritoneal cavity of the patient, once the connector at the other end of the peritoneal catheter 24 is contaminated when the peritoneal dialysis device is being installed or replaced, a new operation must be performed to replace the contaminated peritoneal catheter 24 with a new peritoneal catheter 24 for implantation in the patient, which brings unnecessary risk and workload. In order to avoid possible contamination when the connecting of the injection catheter 20 to the peritoneal catheter 24, in one embodiment of the present invention, the injection catheter connector 21 is connected to the peritoneal catheter 24 through an isolating short tube 25, such that the injection catheter connector 21 is prevented from being connected directly to the peritoneal catheter 24. Therefore, when connecting the injection catheter 20, only the isolating short tube 25 will be contacted, thus preventing the injection catheter 20 or the care worker from directly contacting the peritoneal catheter. Even if contamination occurs, one only need to replace the isolating short tube 25 without having to re-surgically replace the peritoneal catheter 24. In the peritoneal dialysis device for animals 1 shown in Fig. 1, the injection catheter connector 21 is a male connector, and both ends of the isolating short tube 25 are female connectors, and the connector of the peritoneal catheter 24 contacting the isolating short tube is a male connector. As the connector of the injection catheter 21 and the connector of the peritoneal catheter 24 contacting the isolating short tube are both male connectors, therefore the two connectors cannot be connected directly, thus preventing cross-contamination. The injection catheter connector 21 may also be a female connector, and the connectors at both ends of the isolating short tube 25 may also be male connectors, and the connector of the peritoneal catheter 24 contacting the isolating short tube may be a female connector. However, which one is a male connector or a female connector, is merely an embodiment of the present invention, and should not be conceived to limit the scope of the present patent application.

In one embodiment of the present invention, the injection catheter 20 further comprises an injection catheter clamp 22 disposed on the injection catheter 20 and configured to open or close the passageway of the injection catheter 20. However, it may not be necessary to use the catheter clamp 12 to open or close the passageway of the injection catheter 20. The catheter may also be manually squeezed by a machine or a care worker to close the passageway of the injection catheter 20, or controlled by a valve disposed inside the injection catheter 20 to open or close the passageway of the catheter 20 (not shown).

In one embodiment of the present invention, the injection catheter comprises a waterwheel 23 that rotates in response to the flow of fluid in the injection catheter 20. For example, when the passageway of injection catheter 20 is open and the fluid therein is flowing, the fluid pushes the waterwheel 23 to rotate. When the passageway of the injection catheter 20 is closed and the fluid therein is stagnant, the waterwheel 23 cannot be pushed and rotated. Therefore, the care worker may monitor the flow of the fluid in the injection catheter by observing the rotation of the waterwheel 23, thus preventing the stagnation of the dialysate flow.

The waste fluid catheter 30 is used for transporting the waste fluid flowing out from the peritoneal cavity to the direction of the waste fluid container 33. The waste fluid catheter 30 is connected to the waste fluid container 33 through the waste fluid container connector 31. The waste fluid flowing out from the peritoneal cavity sequentially passes through the peritoneal catheter 24, the injection catheter connector 21, the injection catheter 20, the four-way joint 50, the waste fluid catheter 30, and the waste fluid container connector 31 before entering into the waste fluid container 33.

In one embodiment of the present invention, the waste fluid container connector 31 is a luer taper. In the peritoneal dialysis device for animals 1 shown in Fig. 1, the portion of the waste fluid container connector 31 connected to the waste fluid catheter 30 is a male connector, and the portion of the waste fluid container connector 31 connected to the waste fluid container 33 is a female connector. In fact, the portion of the waste fluid container connector 31 connected to the waste fluid catheter 30 may also be a female connector, and the portion of the waste fluid container connector 31 connected to the waste fluid container 33 may also be a male connector. However, which one is male connector or female connector, is merely an embodiment of the invention, and should not be conceived to limit the scope of this patent application.

In one embodiment of the present invention, the waste fluid catheter 30 further comprises a waste fluid catheter clamp 32 disposed on the waste fluid catheter 30 and configured to open or close the passageway of the waste fluid catheter 30. However, it may not be necessary to use the catheter clamp 32 to open or close the passageway of the waste fluid catheter 30. The catheter may also be manually squeezed by a machine or a care worker to close the passageway of the waste fluid catheter 30, or controlled by a valve disposed inside the waste fluid catheter 30 to open or close the passageway of the catheter 30 (not shown). In one embodiment of the present invention, the waste fluid catheter 30 includes a sampling port 34, which allows a doctor or a care worker to sample the waste fluid. The inventor has found that when the sampling port 34 is provided in the injection catheter 20, the sample is diluted by the injected dialysate, because the injection catheter 20 is used for both injecting the dialysate and drawing out the waste fluid. When the sampling port 34 is provided in the waste fluid catheter 30, the sample will not be diluted by the dialysate and the sampling will be more accurate, because the waste fluid catheter 30 is only used for transporting the waste fluid.

The syringe catheter 40 is used for transporting the dialysate in the four-way joint 50 to the syringe 41, and transporting the dialysate flowing out from the syringe 41 to the four-way joint 50. The barrel of the syringe 41 is marked with a scale for quantifying the amount of dialysate injected.

In the peritoneal dialysis device for animals 1 of the present invention, the dialysate is drawn up into the syringe 41 before injected into the peritoneum, and the amount of the dialysate is precisely quantified on the scale of the syringe 41, to overcome the low accuracy of the conventional peritoneal dialysis device. Then the care worker or machine slowly but stably injects the dialysate in the syringe 41 through the catheters and connectors into the peritoneal cavity by applying a steady force to the plunger of the syringe 41, to overcome the instability problem of conventional peritoneal dialysis devices which rely on gravity to push the fluid in the catheter. When the syringe 41 is installed on a machine which can apply a stable force to push the plunger of the syringe slowly and at a constant speed, the peritoneal dialysis device for animals according to the present invention can automatically inject the dialysate without the need for human care workers to perform and supervise personally, thus saving a lot of manpower.

One embodiment of the method for using the peritoneal dialysis device for animals of the present invention

Step S100: The dialysate is drawn up and the amount of the dialysate to be injected is quantified. The machine or care worker closes the passageway of the injection catheter 20 and the waste fluid catheter 30 by means of the catheter clamps 22, 32, valves, or directly squeezing the catheter, and pulls the plunger of the syringe 41 to create negative pressure to draw up the dialysate out of the dialysate container. The dialysate flowing out from the dialysate container sequentially passes through the dialysate container connector 11, the dialysate catheter 10, the four-way joint 50 and the syringe catheter 41 before entering into the syringe 41.

Step S200: The dialysate is injected into the peritoneal cavity of the patient. The machine or care worker closes the passageway of the dialysate catheter 10 and the waste fluid catheter 30 by means of the catheter clamps 12, 32, valves or directly squeezing the catheter, and opens the passageway of the injection catheter 20 by means of the catheter clamp 22, a valve or directly releasing the catheter and pushes the plunger of the syringe 41 to force the dialysate in the syringe 41 to flow out. The dialysate flowing out from the syringe 41 sequentially passes through the syringe catheter 40, the four-way joint 50, the injection catheter 20, the injection catheter connector 21, and the peritoneal catheter 24 before entering into the peritoneal cavity of the patient, allowing the dialysate to contact the patient's peritoneum for a period of time. Using the peritoneum as a dialysis semi-permeable membrane, the body wastes are removed and transported to the dialysate of the peritoneal cavity.

Step S300: The waste fluid containing body wastes from the patient's peritoneal cavity is drained to the waste fluid container 33. The machine or care worker opens the passageway of the waste fluid catheter 30 by means of the catheter clamp 32, a valve, or directly releasing the catheter. As the dialysate catheter 10 is already closed, and the plunger of the syringe 41 has been pushed down to the bottom, so the pressure in the peritoneal cavity pushes the waste fluid containing body wastes toward the direction of the waste fluid container 33. However, whether the waste fluid is discharged and the amount of the waste fluid discharged may also be controlled by pulling/pushing of the syringe by the care worker. The waste fluid flowing out from the peritoneal cavity sequentially passes through the peritoneal catheter 24, the injection catheter connector 21, the injection catheter 20, the four-way joint 50, the waste fluid catheter 30, and the waste fluid container connector 31 before entering into the waste fluid container 33.

According to the structure and method described above, the object of the present invention is to provide a peritoneal dialysis device for animals which can (1) accurately quantify the amount of the dialysate injected, (2) slowly but continuously and stably inject the dialysate, (3) automatically inject the dialysate, and (4) accurately quantify the amount of the waste fluid discharged, without the need for human care workers to perform and supervise personally, thus saving a lot of manpower.

To sum up, the embodiments of the present invention are disclosed as above, but the above embodiments are not intended to limit the present invention. Those of ordinary skill in the art may make various modifications without departing from the spirit and scope of the present disclosure. So the protection scope of the invention should only be defined by the appended claims.

## Claims

1. A peritoneal dialysis device for animals, comprising:
a dialysate catheter, one end of which is connected to a dialysate container connector and the other end of which is connected to a four-way joint;
an injection catheter, one end of which is connected to an injection catheter connector and the other end of which is connected to the four-way joint;
a waste fluid catheter, one end of which is connected to a waste fluid container connector and the other end of which is connected to the four-way joint; and
a syringe catheter, one end of which is connected to a syringe and the other end of which is connected to the four-way joint.

2. The peritoneal dialysis device for animals according to claim 1, wherein
the dialysate catheter is connected to a dialysate container through the dialysate container connector;
the injection catheter is connected to a peritoneal catheter through the injection catheter connector; and
the waste fluid catheter is connected to a waste fluid container through the waste fluid container connector.

3. The peritoneal dialysis device for animals according to claim 1, wherein each of the dialysate container connector, the injection catheter connector and the waste fluid container connector is a luer taper.

4. The peritoneal dialysis device for animals according to claim 1, wherein the injection catheter comprises a waterwheel that rotates in response to the flow of fluid in the injection catheter.

5. The peritoneal dialysis device for animals according to claim 1, wherein the waste fluid catheter comprises a sampling port.

6. The peritoneal dialysis device for animals according to claim 1, wherein
the dialysate catheter comprises a dialysate catheter clamp disposed on the dialysate catheter, and the dialysate catheter clamp is configured to open or close the passageway of the dialysate catheter;
the injection catheter comprises an injection catheter clamp disposed on the injection catheter, and the injection catheter clamp is configured to open or close the passageway of the injection catheter; and
the waste fluid catheter comprises a waste fluid catheter clamp disposed on the waste fluid catheter, and the waste fluid catheter clamp is configured to open or close the passageway of the waste fluid catheter.

7. The peritoneal dialysis device for animals according to claim 1, wherein
the dialysate catheter comprises a multi-way joint including three or more ends, wherein two or more ends are connected to a plurality of the dialysate container connectors, and the other one end is connected to the four-way joint.

8. The peritoneal dialysis device for animals according to claim 7, wherein
the dialysate catheter is connected to a plurality of dialysate containers through the plurality of the dialysate container connectors.

9. The peritoneal dialysis device for animals according to claim 2, wherein
the injection catheter connector is connected to the peritoneal catheter through an isolating short tube, so as to prevent the injection catheter connector from being directly connected to the peritoneal catheter.
